(19) Europäisches Patentamt
European Patent Office
Office européen des brevets

(11) Veröffentlichungsnummer : **0 014 674**
**B1**

(12) # EUROPÄISCHE PATENTSCHRIFT

(45) Veröffentlichungstag der Patentschrift : **05.10.83**

(51) Int. Cl.³ : **C 07 C127/22**, C 07 C 93/14, A 01 N 47/34

(21) Anmeldenummer : **80810020.0**

(22) Anmeldetag : **28.01.80**

(54) **Substituierte N-Phenyl-N'-benzoylharnstoffe, Verfahren zu ihrer Herstellung, diese Verbindungen enthaltende Mittel und ihre Verwendung zur Bekämpfung von Schädlingen; substituierte 4-(Propenyloxy)-aniline.**

(30) Priorität : **01.02.79 CH 982/79**

(43) Veröffentlichungstag der Anmeldung : **20.08.80 Patentblatt 80/17**

(45) Bekanntmachung des Hinweises auf die Patenterteilung : **05.10.83 Patentblatt 83/40**

(84) Benannte Vertragsstaaten : **AT BE CH DE FR GB IT NL**

(56) Entgegenhaltungen :
**JOURNAL OF AGRICULTURAL AND FOOD CHEMISTRY, Band 21, Nr. 3, Mai/Juni 1973, Seiten 348-354 American Chemical Society N.W. WASHINGTON DC, U.S. A.K. WELLINGA et al. : « Synthesis and Laboratory Evaluation of 1-(2,6-Disubstituted benzoyl)-phenyl-ureas, a New Class of Insecticides. I. 1-(2,6-Dichlorobenzoyl)-phenylureas »**

(73) Patentinhaber : **CIBA-GEIGY AG**
**Patentabteilung Postfach**
**CH-4002 Basel (CH)**

(72) Erfinder : **Ehrenfreund, Josef, Dr.**
**Langenhagweg 11**
**CH-4123 Allschwil (CH)**

**EP 0 014 674 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Substituierte N-Phenyl-N'-benzoylharnstoffe, Verfahren zu ihrer Herstellung, diese Verbindungen enthaltende Mittel und ihre Verwendung zur Bekämpfung von Schädlingen ; substituierte 4-(Propenyloxy)-aniline

Die vorliegende Erfindung betrifft neue substituierte N-4-(3-Bromallyloxy)-phenyl-N'-benzoylharnstoffe, Verfahren zu ihrer Herstellung und ihre Verwendung in der Schädlingsbekämpfung.

Die erfindungsgemässen substituierten N-4-(3-Bromallyloxy)-phenyl-N'-benzoylharnstoffe haben die Formel I

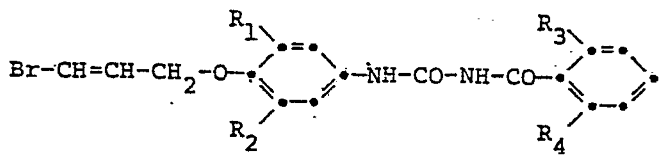

(I)

worin

$R_1$ und $R_2$ unabhängig voneinander Wasserstoff, Chlor oder Brom ;
$R_3$ Fluor oder Chlor ; und
$R_4$ Wasserstoff, Fluor oder Chlor

bedeuten.

Wegen ihrer Wirkung als Schädlingsbekämpfungsmittel bevorzugt sind erfindungsgemässe Verbindungen der Formel I, die dadurch gekennzeichnet sind, dass $R_1$ und $R_2$ Chlor oder Brom, $R_3$ Fluor oder Chlor und $R_4$ Wasserstoff, Chlor oder Fluor bedeuten. Besonders wirksam sind die erfindungsgemässen Verbindungen der Formel I, bei denen $R_3$ und $R_4$ Fluor darstellen.

Die Verbindungen der Formel I fallen als cis/trans-Isomerengemische an. In diesem Sinne sind als Verbindungen der Formel I sowohl die cis- oder trans-Formen als auch die entsprechenden Isomerengemische zu verstehen. Ein Isomerengemisch kann z. B. mit Hilfe der bekannten chromatographischen Trennmethoden und anschliessender Eluierung in die isomeren Formen getrennt werden. Zur Synthese stereochemisch einheitlicher Verbindungen der Formel I verwendet man mit Vorteil stereochemisch einheitliche Ausgangsverbindungen der nachstehenden Formeln II oder IV.

Die Verbindungen der Formel I können nach an sich bekannten Verfahren hergestellt werden (vgl. u. a. die deutschen Offenlegungsschriften Nr. 2.123.236, 2.601.780).

So kann man z. B. eine Verbindung der Formel I erhalten durch Umsetzung

a) einer Verbindung der Formel II

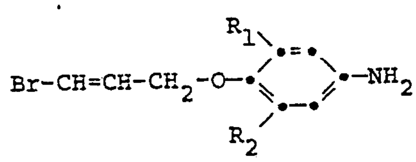

(II)

mit einer Verbindung der Formel III

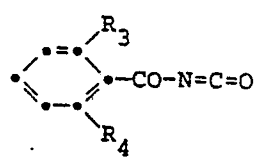

(III)

oder

b) einer Verbindung der Formel IV

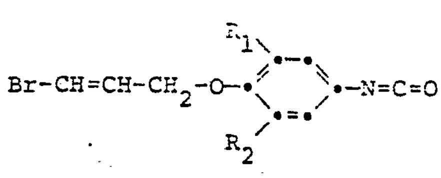

(IV)

gegebenenfalls in Gegenwart einer basischen Substanz mit einer Verbindung der Formel V

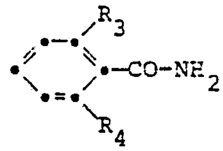

(V)

In den obigen Formeln II, III, IV und V haben die Reste $R_1$, $R_2$, $R_3$, und $R_4$ die unter Formel I vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z. B. Aether und ätherartige Verbindungen, wie Diäthyläther, Dipropyläther, Dibutyläther, Dioxan, Dimethoxyäthan und Tetrahydrofuran ; N,N-dialkylierte Carbonsäureamide ; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol ; Nitrile, wie Acetonitril oder Propionitril ; Dimethylsulfoxid sowie Ketone, z. B. Aceton, Methyläthylketon, Methylisopropylketon und Methylisobutylketon. Verfahren a) wird im allgemeinen bei einer Temperatur von – 10 bis 100 °C, vorzugsweise zwischen 15 und 25 °C, gegebenenfalls in Gegenwart einer organischen Base, z. B. Triäthylamin, durchgeführt. Die Durchführung von Verfahren b) erfolgt bei einer Temperatur von 0 bis 150 °C, vorzugsweise beim Siedepunkt des verwendeten Lösungsmittels, gegebenenfalls in Gegenwart einer organischen Base, wie Pyridin, und/oder unter Zusatz eines Alkali- oder Erdalkalimetalls, vorzugsweise Natrium.

Die Ausgangsstoffe der Formeln II, III, IV und V sind bekannt oder können, falls sie neu sind, analog bekannten Verfahren hergestellt werden. Man kann z. B. die bisher noch nicht bekannten Verbindungen der Formel II durch Reaktion des entsprechend substituierten p-Hydroxy-acetanilids mit 1,3 Dibrompropen und anschliessende saure Hydrolyse des erhaltenen Umsetzungsproduktes herstellen :

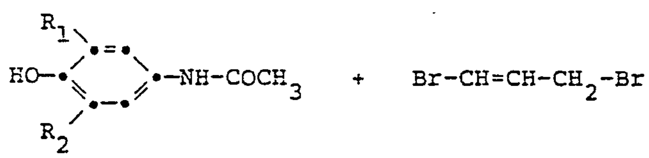

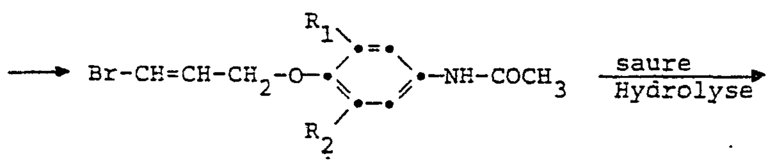


(II)

In obigen Formeln haben $R_1$ und $R_2$ die unter Formel I angegebenen Bedeutungen. Die neuen Ausgangsverbindungen, welche zu den wertvollen Schädlingsbekämpfungsmitteln der Formel I führen, bilden ebenfalls einen Gegenstand der vorliegenden Erfindung.

Es ist bereits bekannt, dass bestimmte N-Phenyl-N'-benzoylharnstoffe insektizide Eigenschaften besitzen (vgl. deutsche Offenlegungsschriften Nr. 2.123.236, 2.504.982, 2.537.413, 2.601.780, 2.726.684 und 2.820.696, die belgischen Patentschriften 832.304, 843.906 und 844.066 sowie die US-Patentschrift 4.089.975).

Ueberraschenderweise wurde nun gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen : Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer Wirkung gegenüber Fliegen, wie z. B. Musca domestica, und Mückenlarven können Verbindungen der Formel I auch zur Bekämpfung von pflanzenschädigenden Frassinsekten in Zier- und Nutzpflanzen, insbesondere in Baumwollkulturen (z. B. gegen Spodoptera littoralis und Heliothis virescens), sowie in Obst- und Gemüsekulturen (z. B. gegen Leptinotarsa decemlineata, Laspeyresia pomonella und Epilachna varivestis) eingesetzt werden. Hierbei zeichnen sich die Verbindungen der Formel I vor allem durch ausgeprägte larvizide sowie ovolarvizide Wirksamkeit aus. Ausserdem weisen einige Verbindungen auch gute ovizide Wirkung gegen Insekten auf. Werden Verbindungen der Formel I von adulten Insekten mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindung der Formel I eignen sich weiterhin zur Bekämpfung von Ektoparasiten an Haus- und Nutztieren, z. B., durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z. B. folgende Wirkstoffe in Betracht :

organische Phosphorverbindungen,

Nitrophenole und Derivate,
Formamidine, Harnstoffe
Carbamate und
chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u. a. : Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind « cattle dips », d. h. Viehbäder, und « spray races », d. h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden :

Feste Aufarbeitungsformen : Stäubemittel, Streumittel, Granulate (Umhüllungsgranulate, Imprägnierungsgranulate und Homogengranulate) ;

Flüssige Aufarbeitungsformen :

a) in Wasser dispergierbare Wirkstoffkonzentrate : Spritzpulver (wettable powders), Pasten, Emulsionen ;

b) Lösungen.

Der Gehalt an Wirkstoff in den oben beschriebenen Mitteln liegt zwischen 0,1 bis 95 %.

Die Wirkstoffe der Formel I können beispielsweise wie folgt formuliert werden :

Stäubemittel : Zur Herstellung eines a) 5 %igen und b) 2 %igen Stäubemittels werden die folgenden Stoffe verwendet :

a) 5 Teile Wirkstoff,
95 Teile Talkum ;

b) 2 Teile Wirkstoff,
1 Teil hochdisperse Kieselsäure,
97 Teile Talkum.

Die Wirkstoffe werden mit den Trägerstoffen vermischt und vermahlen.

Granulat : Zur Herstellung eines 5 %igen Granulates werden die folgenden Stoffe verwendet :

5 Teile Wirkstoff,
0,25 Teile epoxydiertes Pflanzenöl,
0,25 Teile Cetylpolyglykoläther,
3,50 Teile Polyäthylenglykol,
91 Teile Kaolin (Korngrösse 0,3-0,8 mm).

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver : Zur Herstellung eines a) 40 %igen, b) und c) 25 %igen d) 10 %igen Spritzpulvers werden folgende Bestandteile verwendet :

a) 40 Teile Wirkstoff,
5 Teile Ligninsulfonsäure-Natriumsalz,
1 Teil Dibutylnaphthalinsulfonsäure-Natriumsalz,
54 Teile Kieselsäure ;

b) 25 Teile Wirkstoff,
4,5 Teile Calcium-Ligninsulfonat,
1,9 Teile Champagne-Kreide/Hydroxyäthylcellulose-Gemisch (1 : 1),
1,5 Teile Natrium-dibutyl-naphthalinsulfonat,
19,5 Teile Kieselsäure,

19,5 Teile Champagne-Kreide,
28,1 Teile Kaolin;
c) 25 Teile Wirkstoff,
2,5 Teile Isooctylphenoxy-polyoxyäthylen-äthanol,
1,7 Teile Champagne-Kreide/Hydroxyäthylcellulose-Gemisch (1 : 1),
8,3 Teile Natrimaluminiumsilikat,
16,5 Teile Kieselgur,
46 Teile Kaolin;
d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentratin verdünnen lassen.

Emulgierbare Konzentrate: Zur Herstellung eines a) 10 %igen b) 25 %igen und c) 50 %igen emulgierbaren Konzentrates werden folgende Stoffe verwendet:

a) 10 Teile Wirkstoff,
3,4 Teile epoxydiertes Pflanzenöl,
3,4 Teile eines Kombinationsemulgators, bestehend aus Fettalkoholpolyglykoläther und Alkylaralkylsulfonat-Calcium-Salz,
40 Teile Dimethylformamid,
43,2 Teile Xylol;
b) 25 Teile Wirkstoff,
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol;
c) 50 Teile Wirkstoff,
4,2 Teile Tributylphenol-Polyglykoläther,
5,8 Teile Calcium-Dodecylbenzolsulfonat,
20 Teile Cyclohexanon,
20 Teile Xylol.

Aus solchen Konzentrationen können durch Verdünnen mit Wasser Emulsionen jeder gewünschten Konzentration hergestellt werden.

Sprühmittel: Zur Herstellung eines a) 5 %igen und b) 95 %igen Sprühmittels werden die folgenden Bestandteile verwendet:

a) 5 Teile Wirkstoff,
1 Teil epoxydiertes Pflanzenöl,
94 Teile Benzin (Siedegrenzen 160°-190 °C);
b) 95 Teile Wirkstoff,
5 Teile epoxydiertes Pflanzenöl.

Beispiel 1

Zu einer Lösung von 6 g 3,5-Dichlor-4-(3'-bromprop-2-enyloxy)-anilin in 25 ml absolutem Aether werden bei Raumtemperatur und unter Feuchtigkeitsausschluss 3,8 g 2,6-Difluorbenzoylisoc yanat zugegeben. Der nach kurzer Zeit ausfallende Niederschlag wird abgesaugt und mit Aether gewaschen. Durch Umkristallisation aus Toluol erhält man analysenreinen N-3,5-Dichlor-4-(3'-bromprop-2-enyloxy)-phenyl-N'-(2,6-difluor)-benzoylharnstoff vom Schmelzpunkt 150-160 °C (als Gemisch der beiden möglichen Stereoisomeren).

Analog den vorstehend beschriebenen Arbeitsweisen werden die folgenden Verbindungen der Formel I hergestellt:

(Siehe Tabelle Seite 6 f.)

| $R_1$ | $R_2$ | $R_3$ | $R_4$ | Schmelzpunkt [°C] |
|---|---|---|---|---|
| H | H | F | F | 155-160 |
| Cl | Cl | Cl | H | 159-168 |
| H | H | Cl | H | 148-165 |
| Cl | Cl | F | F | 150-160 |
| Br | Cl | F | H | 158-160 |
| Br | Cl | F | Cl | |
| Br | Cl | F | F | 175-181 |
| Br | Cl | Cl | H | 184-190 |
| Br | Br | F | H | 160-162 |
| Br | Br | F | Cl | 162-164 |
| Br | Br | F | F | 176-185 |
| Br | Br | Cl | H | 186-188 |
| Cl | Cl | F | H | 174-176 |
| Cl | Cl | Cl | Cl | 182-193 |
| Br | Br | Cl | Cl | 193-195 |
| Br | Cl | Cl | Cl | 163-165 |
| Cl | Cl | F | Cl | 130-132 |

## Beispiel 2

Wirkung gegen Musca domestica :

Je 50 frisch zubereites CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dan wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss Beispiel 1 zeigten gute Wirkung im obigen Test.

## Beispiel 3

Wirkung gegen Lucilia sericata :

Zu 9 ml eines Zuchtmediums wurden bei 50 °C 1 ml einer 0,5 % Aktivsubstanz enthaltenden wässrigen Lösung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss Beispiel 1 zeigten in diesem Test gute Wirkung gegen Lucilia sericata.

Beispiel 4

Wirkung gegen Aëdes aegypti :

Auf die Oberfläche von 150 ml Wasser, das sich in einem Behälter befindet, wurde so viel einer 0,1 %igen acetonischen Lösung des Wirkstoffes pipettiert, dass Konzentrationen von je 10, 5 und 1 ppm erhalten wurden. Nach Verdunsten des Acetons wurde der Behälter mit 30-40 2-tägigen Aëdes-Larven beschickt. Nach 1, 2 und 5 Tagen wurde die Mortälität geprüft.

Verbindungen gemäss Beispiel 1 zeigten in diesem Test gute Wirkung gegen Aëdes aegypti.

Beispiel 5

Wirkung gegen Spodoptera littoralis und Heliothis virescens (Larven ; Frass- und Kontaktwirkung) :

Eingetopfte ca. 30 cm hohe Baumwoll- bzw. Soja-Pflanzen wurden mit einer verdünnten, wässrigen Emulsions-Zubereitung des zu prüfenden Wirkstoffes bis zum Abtropfen besprüht. Nach Antrocknen des Spritzbelages wurden die Baumwoll-Pflanzen mit je 5 Larven im dritten Larvalstadium von Spodoptera und die Soja-Pflanzen mit je 10 Larven im dritten Larvalstadium von Heliothis besetzt. Die Ansätze wurden 5 Tage bei künstlichem Licht, einer Temperatur von etwa 26 °C und 50-60 % relativer Luftfeuchtigkeit gehalten.

Die Auswertung erfolgte hinsichtlich prozentualer Mortalität, Frasshemmung, Deformationen und Entwicklungshemmungen im Vergleich zu unbehandelten Kontrollen.

Die Verbindungen gemäss Beispiel 1 zeigten in diesem Test gute Wirkung.

Beispiel 6

Wirkung gegen Epilachna varivestis (Larven) :

Etwa 15-20 cm hohe Phaseolus vulgaris-Pflanzen (Buschbohnen) wurden mit einer wässrigen, den zu prüfenden Wirkstoff enthaltenden Emulsions-Zubereitung besprüht. Nach dem Antrocknen des Sprühbelages wurden pro Pflanze 10 Larven von Epilachna varivestis (Mexikanischer Bohnenkäfer) im 4. larvalen Stadium angesetzt. Ueber die behandelten Pflanzen wurde ein Pflastikzylinder gestülpt, der mit einem Kupfer-Gazedeckel abgedeckt war.

Nach 1 und 2 Tagen wurde die akute Wirkung (% Mortalität) bestimmt. Zur Auswertung hinsichtlich allfälligem Frass-Schaden (Antifeeding-Effekt), Entwicklungs- und Häutungsstörungen wurden die Versuchstiere während weiterer 3 Tage beobachtet.

Verbindungen gemäss Beispiel 1 zeigten gute Wirkung in obigem Test.

Beispiel 7

Wirkung gegen Leptinotarsa decemlineata (Larven) :

15 cm hohe in Kulturgefässen befindliche Kartoffelpflanzen, wurden mit einer wässerigen Emulsions-Zubereitung, enthaltend den zu prüfenden Wirkstoff in einer Konzentration von 500 ppm, gleichmässig bis zur Tropfnässe mit einer Druckluftspritze besprüht. Nach dem Trocknen der Pflanzen, d. h. nach etwa 1,5 Stdn., wurde über dieselben ein Plastikzylinder gestülpt, und pro Pflanze wurden je 10 Kartoffelkäferlarven des 3. Stadiums angesetzt. Die Zylinder wurden dann mit einem Kupfergaze-Deckel abgeschlossen und in der Dunkelheit bei 28 °C und 60 % rel. Luftfeuchtigkeit stehen gelassen.

Nach 1, 2 und 8 Tagen wurde auf Mortalität (Rückenlage) und % — Frass-Schaden an den Pflanzen geprüft.

Verbindungen gemäss Beispiel 1 zeigten gute Wirkung in obigem Test.

Beispiel 8

Chemosterilisierende Wirkung auf Anthonomus grandis.

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlegen in ein wässriges Desinfektionsmittel (wie z. B. « Actamer B 100 ») desinfiziert und dann in Schalen, die eine geeignete

Larvaldiät enthielten, deponiert. Nach 7 Tagen wurde untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäss Beispiel 1 zeigten gute Wirkung in obigem Test.

**Ansprüche**

1. Verbindung der Formel I

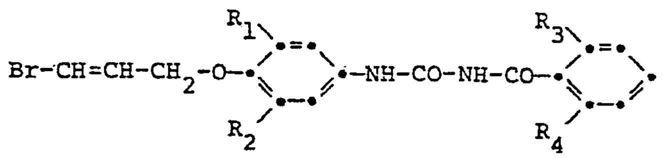

(I)

worin

$R_1$ und $R_2$ unabhängig voneinander Wasserstoff, Chlor oder Brom ;

$R_3$ Fluor oder Chlor ; und

$R_4$ Wasserstoff, Fluor oder Chlor

bedeuten.

2. Verbindung der Formel I gemäss Anspruch 1, dadurch gekennzeichnet, dass

$R_1$ und $R_2$ Chlor oder Brom ;

$R_3$ Fluor oder Chlor ; und

$R_4$ Wasserstoff, Chlor oder Fluor bedeuten.

3. Verbindung der Formel I gemäss Anspruch 1 oder 2, dadurch gekennzeichnet, dass $R_3$ und $R_4$ Fluor bedeuten.

4. Verbindung gemäss Anspruch 3 der Formel

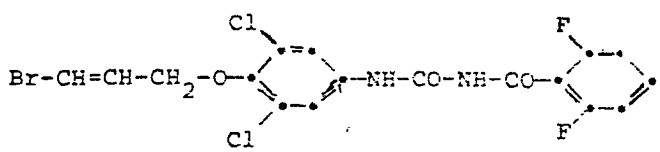

5. Verbindung gemäss Anspruch 1 der Formel

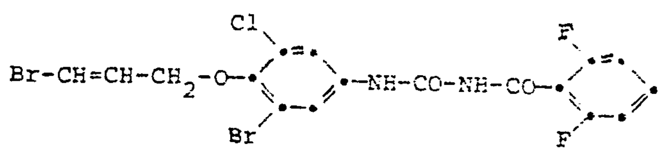

6. Verbindung gemäss Anspruch 3 der Formel

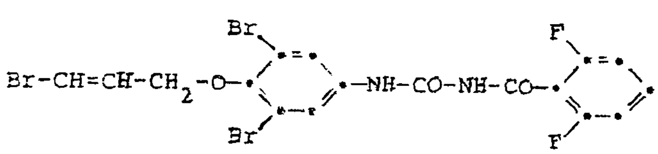

7. Verfahren zur Herstellung einer Verbindung gemäss den Ansprüchen 1 bis 6, dadurch gekennzeichnet, dass man

a) eine Verbindung der Formel II

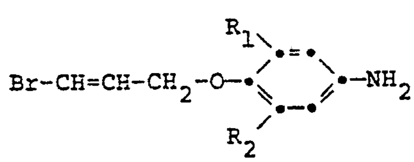

(II)

mit einer Verbindung der Formel III

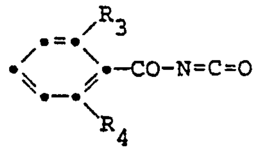

(III)

oder

b) eine Verbindung der Formel IV

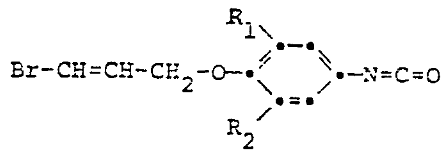

(IV)

mit einer Verbindung der Formel V

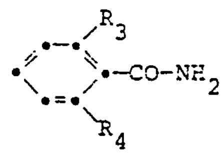

(V)

umsetzt, wobei in den Formeln II bis V die Reste $R_1$, $R_2$, $R_3$ und $R_4$ die in der Ansprüchen 1 bis 3 angegebenen Bedeutungen haben.

8. Schädlingsbekämpfungsmittel, welches als aktive Komponente eine Verbindung gemäss den Ansprüchen 1 bis 6 zusammen mit geeigneten Trägern und/oder anderen Zuschlagstoffen enthält.

9. Verwendung einer Verbindung gemäss den Ansprüchen 1 bis 6 zur Bekämpfung von Schädlingen, vorzugsweise von larvalen Insektenstadien.

10. Verbindung der Formel II

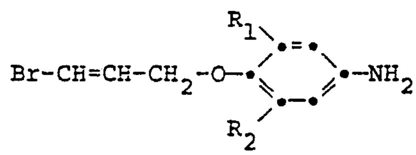

(II)

wobei die Reste $R_1$ und $R_2$ die in Anspruch 1 angegebenen Bedeutungen haben.

**Claims**

1. A compound of the formula I

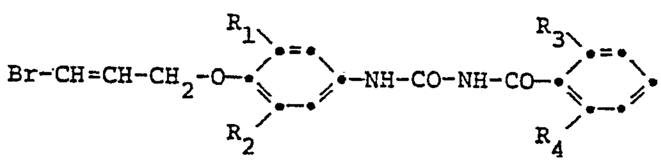

(I)

wherein

$R_1$ and $R_2$ independently of one another are each hydrogen, chlorine or bromine,
$R_3$ is fluorine or chlorine, and
$R_4$ is hydrogen, fluorine or chlorine.

2. A compound of the formula I according to Claim 1, wherein
$R_1$ and $R_2$ are each chlorine or bromine,
$R_3$ is fluorine or chlorine, and
$R_4$ is hydrogen, chlorine or fluorine.

3. A compound of the formula I according to Claim 1 or 2, wherein $R_3$ and $R_4$ are each fluorine.

4. A compound according to Claim 3 of the formula

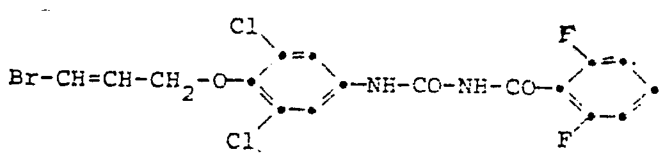

5. A compound according to Claim 1 of the formula

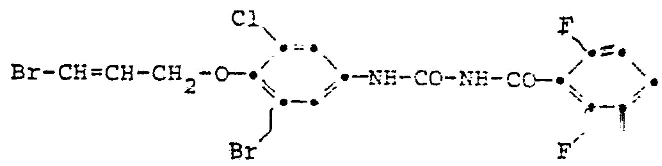

6. A compound according to Claim 3 of the formula

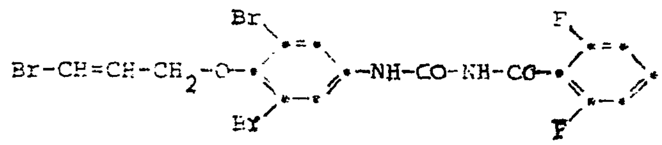

7. A process for producing a compound according to Claims 1 to 6 inclusive, which process comprises reacting

a) a compound of the formula II

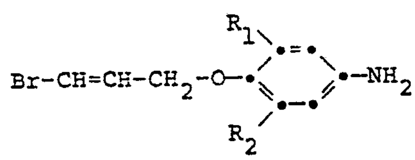 (II)

with a compound of the formula III

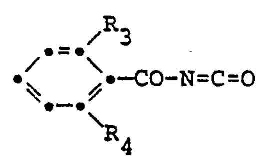 (III)

or

b) a compound of the formula IV

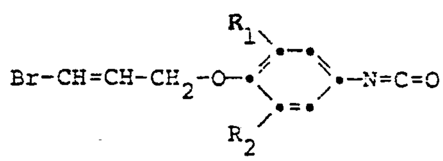 (IV)

with a compound of the formula V

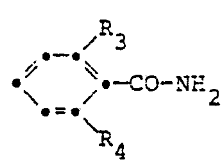 (V)

where in the formulae II to V the radicals $R_1$, $R_2$, $R_3$ and $R_4$ have the meanings given in Claims 1 to 3.

8. A pesticidal composition which contains as active ingredient a compound according to Claims 1 to 6 inclusive, together with suitable carriers and/or other additives.

9. Use of a compound according to Claims 1 to 6 inclusive for combating pests, particularly larval insect stages.

10. A compound of the formula II

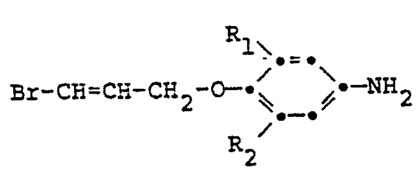 (II)

wherein the radicals $R_1$ and $R_2$ have the meanings defined in Claim 1.

**Revendications**

1. Composé de formule I

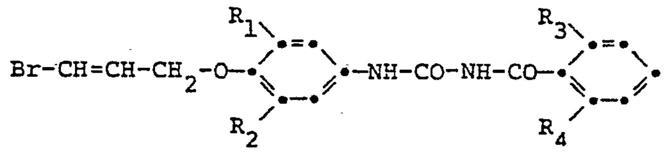

(I)

dans laquelle

$R_1$ et $R_2$ représentent chacun, indépendamment l'un de l'autre, l'hydrogène, le chlore ou le brome,
$R_3$ représente le fluor ou le chlore, et
$R_4$ représente l'hydrogène, le fluor ou le chlore.

2. Composé de formule I selon la revendication 1, caractérisé en ce que
$R_1$ et $R_2$ représentent le chlore ou le brome,
$R_3$ représente le fluor ou le chlore,
$R_4$ représente l'hydrogène, le chlore ou le fluor.

3. Composé de formule I selon la revendication 1 ou 2, caractérisé en ce que $R_3$ et $R_4$ représentent le fluor.

4. Composé selon la revendication 3 de formule

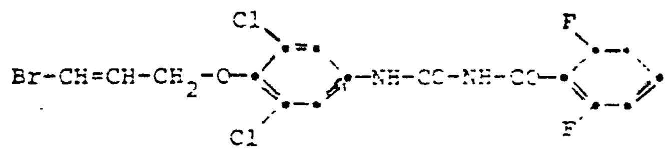

5. Composé selon la revendication 1 de formule

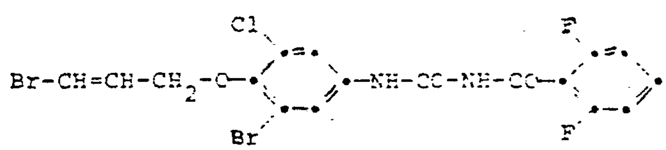

6. Composé selon la revendication 3 de formule

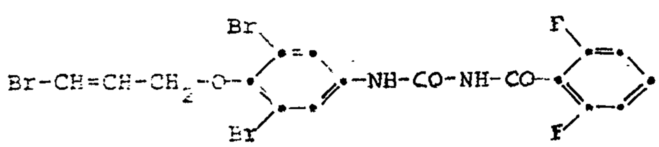

7. Procédé de préparation d'un composé selon l'une des revendications 1 à 6, caractérisé en ce que :
a) on fait réagir un composé de formule II

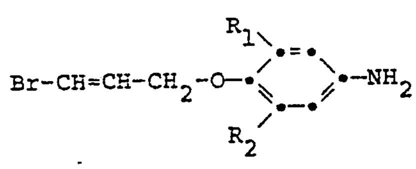

(II)

avec un composé de formule III

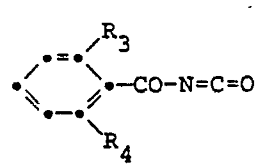

(III)

ou bien

b) on fait réagir un composé de formule IV

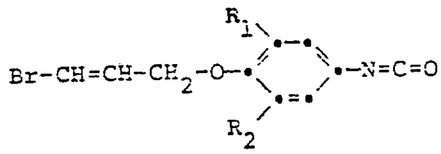

(IV)

avec un composé de formule V

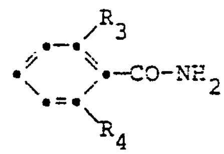

(V)

les symboles $R_1$, $R_2$, $R_3$ et $R_4$ des formules II à V ayant les significations indiquées dans les revendications 1 à 3.

8. Produit pesticide contenant en tant que composant actif un composé selon l'une des revendications 1 à 6 avec des véhicules et/ou d'autres additifs appropriés.

9. Utilisation d'un composé selon l'une des revendications 1 à 6, dans la lutte contre les parasites, de préférence les stades larvaires des insectes.

10. Composé de formule II

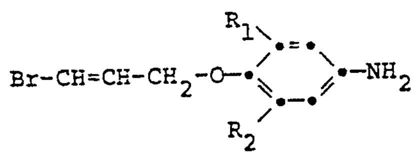

(II)

dans laquelle les symboles $R_1$ et $R_2$ ont les significations indiquées dans la revendication 1.